# EUROPEAN PATENT SPECIFICATION

(11) **EP 4 421 064 B1**
(45) Date of publication and mention of the grant of the patent: **01.10.2025**
(21) Application number: 24159151.0
(22) Date of filing: 22.02.2024
(51) Int. Cl.: C07C 403/24, C09B 61/00, A23L 5/44

(54) **A PREPARATION METHOD OF CAROTENOID AGENT**
VERFAHREN ZUR HERSTELLUNG EINES CAROTINOIDMITTELS
PROCÉDÉ DE PRÉPARATION D'UN AGENT CAROTÉNOÏDE

(30) Priority: 22.02.2023 CN 202310152079
(43) Date of publication of application: 28.08.2024
(73) Proprietor: Zhejiang Keming Biopharmaceutical Co., Ltd, Xinchang, Zhejiang 312500 (CN); ZheJiang Medicine Co., Ltd. Xinchang Pharmaceutical Factory, Xinchang, Zhejiang 312500 (CN)
(72) Inventor: ZHOU, Di, Xinchang, 312500 (CN); XU, Xinde, Xinchang, 312500 (CN); BAI, Yalong, Xinchang, 312500 (CN); YU, Qinghua, Xinchang, 312500 (CN); ZHU, Lanfang, Xinchang, 312500 (CN); WANG, Yongqi, Xinchang, 312500 (CN)
(74) Representative: Casalonga

(56) References cited:
- EP-A1- 3 020 396
- EP-A2- 0 807 431
- DE-T5- 112021 000 453
- US-A1- 2012 018 912

## Description

### Technical Field

The present disclosure relates to the technical field of carotenoid agents, specifically to a preparation method of carotenoid agent.

### Background

Carotenoids are widely found in nature, and their important representative substances are β-carotenoids, astaxanthin, canthaxanthin, and lutein. These substances are widely used as nutrient supplements and colorants in the fields of food, cosmetics, feed, and pharmaceutical industries. Some of them are also precursors of vitamin A, which have the function of vitamin A. Carotenoid is an important substance in the feed industry. They not only can participate in the physiological metabolism of animal bodies, but also can give more vibrant colors to animal skins or feathers, etc., giving people a sense of beauty. Pigments such as lutein, zeaxanthin, and canthaxanthin are widely used in laying hens, broiler chickens, and laying ducks industry, which can make the skins of poultries and the yolks of poultry eggs appear yellow and red, respectively, which are more preferred by people, while astaxanthin is widely found in some marine fishes such as salmons and singmons, etc., as well as in crabs and shrimps. Since these carotenoids cannot be synthesized by animals themselves, they must be obtained through feeding on wild plants or algae by animals. Generally, an appropriate amount of carotenoids should be added to feed to supplement the carotenoid levels in industrially produced animals such as fish and poultries, achieving nutritional and coloring purposes.

Carotenoid is insoluble in water and has low solubility in oil. Carotenoid is a class of highly unstable substances that contain at least nine double bonds in its molecular structure. Due to the cis-trans isomerism of these double bonds, it has many isomers. Moreover, due to the presence of double bonds, carotenoid is highly sensitive to light, heat, and oxygen, making them prone to oxidation and decomposition. It is also prone to isomerization reaction while undergoing the oxidation and decomposition, that is, the transformation from highly active all-trans structures to less active cis structures. Such characteristics make it difficult for carotenoids to be directly applied as products. Therefore, in practical production, carotenoids are generally formulated, in which the active ingredients exist in a finely dispersed form and require protective colloids to resist oxidation, thus achieving their applications in various fields.

In order to improve the coloring effect and increase absorbability of carotenoid, the solubility of which is changed in order to expand its application range, and thus it is required to make the carotenoid into preparations for better application in the feed industry. Currently, some studies have introduced the preparation methods of carotenoid agents.

WO/2010/040683 introduces a preparation method of a ready-to-use emulsion, which uses a glycerin-water mixture as protective colloid, and modified starch is added for swelling under heating, then the aqueous phase and the carotenoid oil melt solution are mixed, and subjected to high-speed stirring and high pressure homogenization into the water dispersible emulsion. This method uses the traditional high-temperature melting method, which will accelerate the oxidation and deterioration process of carotenoids, and the content of isomers has changed greatly, so that the obtained emulsion products cannot be preserved for a long time.

WO91/06292 and WO94/19411 introduce a method for preparing a powdered water dispersible carotenoid by the use of a colloid grinder to grind the β-carotenoids into particulates of 2 µm to 10 µm, and then drying. However, the grinding method is obviously of low efficiency, high energy consumption, and it is difficult to achieve a particle size of 1 µm or less for carotenoids.

US5364563 introduces a process for producing powdered carotenoid agents, which comprises mixing the carotenoid and a vegetable oil of a boiling point of more than 200°C to form a suspension, then subjecting the suspension to high-temperature dissolution by passing it through steam at temperature up to 200°C, and mixing the aqueous phase and the oil phase through a homogenizer to obtain an emulsion, and then passing same through a fluidized bed loading with starch for spray drying to obtain the product. However, such a high-temperature heating process will result in a significant loss in content of carotenoid, and the content of the final all-trans forms will be lower, which is only 70%. In addition, the use of high-temperature and high-pressure superheated steam requires expensive equipment, which has high operation requirements, and the technological process is complicated, and the productivity is low.

In the actual production process of a carotenoid preparation, it is found that carotenoids, especially in the dissolution process, will produce a considerable number of cis isomers, ultimately leading to a low content of all-trans carotenoids in the preparation products. However, all-trans isomers have the advantage of high biological activity, with the main cis isomers, i.e. 9-cis and 13-cis having only 30% to 50% of the biological activity of all-trans isomers. Therefore, increasing the content of all-trans isomers in carotenoid agent is of great significance in improving bioavailability.

Patent CN1836652A describes a preparation method of a powdered water dispersible carotenoid, which comprises dissolving the crude crystal of carotenoid in halogenated hydrocarbons or ester solvents containing an antioxidant and an emulsifier, and adding the obtained solution to high-speed stirring ethanol or isopropanol in the form of spraying, making the carotenoid precipitate in an amorphous powder form of less than 2 µm; then filtering the precipitated carotenoid with a filter membrane or a sintered filter rod, washing the filter cake with ethanol or isopropanol, and filtering to dryness; then adding the filter cake to an aqueous solution containing a protective colloid, first beating with stirring and then homogenizing and emulsifying, followed by removing the residual solvents to prepare a water dispersible solution; finally granulating the dispersion by a spraying method, and then fluidization drying to obtain the water dispersible carotenoid agent. This method has the advantages of low content of residual solvent, fast removal, and high efficiency, but the crystal particle size in the obtained powdered carotenoid is from 0.7 µm to 0.9 µm, the loss rate of carotenoids is relatively high, and the content of all-trans isomers is less than 90%.

US2012/018912 A1 describes a method of preparing nano-dispersed high-all-trans-carotenoid microcapsules; EP0807431A2 introduces a process for the manufacture of carotenoid compositions.

It is difficult for the preparation methods reported above to overcome the isomerization reactions that occur easily during the dissolution of carotenoids in an organic solvent, resulting in low content of all-trans isomers and low biological activity in carotenoid feed additive products.

### Summary

The main object of the present disclosure is to provide a preparation method of carotenoid agent, in order to solve the problems that it is difficult for the preparation methods of carotenoids in the prior art to overcome the isomerization reactions that occur easily during the dissolution of carotenoids in an organic solvent, resulting in low content of all-trans isomers and low biological activity in carotenoid feed additive products.

In order to achieve the above object, according to one aspect of the present disclosure, a preparation method of carotenoid agent is provided, which includes: step S1, mixing a suspended organic dispersion phase of carotenoid crystal with a first organic solvent in a spiral coil of a coil heat exchanger to form a mixed solution, with the carotenoid crystal in the suspended organic phase of the carotenoid crystal being dissolved in the first organic solvent to obtain an oil phase matrix of the carotenoid dissolution solution, wherein the temperature inside the spiral coil is from 50°C to 70°C
step S2, mixing the aqueous phase matrix of the colloidal solution with the oil phase matrix of the carotenoid dissolution solution, and performing emulsification and dispersion as well as homogenization treatment successively to obtain a carotenoid nanodispersion, wherein, the aqueous phase matrix of the colloidal solution comprises a polymer protective colloid, a small molecular carrier, a second antioxidant, and water, and the second antioxidant is a water-soluble antioxidant;
step S3, removing the organic solvent from the carotenoid nanodispersion and concentrating the moisture to obtain a carotenoid emulsion; and
step S4, subjecting the carotenoid emulsion to spray granulation and drying successively to obtain the carotenoid agent;
the temperature of the suspended organic dispersion phase of the carotenoid crystal is from 15°C to 30°C, and the temperature of the first organic solvent is from 50 to 70°C;
the residence time of the mixed solution in the coil heat exchanger is from 40 seconds to 60 seconds;
the aspect ratio of the spiral coil is 500-2,000:1; the inner diameter of the spiral coil is from 0.5 cm to 2 cm and the length is from 5 m to 20 m.

Further, the mass content of the carotenoid crystal in the suspended organic dispersion phase of the carotenoid crystal is from 20% to 40%, and preferably the mass content of the all-trans forms in the carotenoid crystal is greater than or equal to 95%.

Further, the mass ratio of the first organic solvent to the carotenoid crystal is 7.5-20:1.

Further, the suspended organic dispersion phase of the carotenoid crystal further comprises a first antioxidant, which is preferably selected from at least one of butylated hydroxytoluene (BHT), butylated hydroxyanisole (BHA), tert-butylhydroquinone (TBHQ), vitamin C palmitate, sodium ascorbate, ethoxyquinoline, dl-α tocopherol, further preferably dl-α tocopherol.

Further, the mass ratio of the first antioxidant to the carotenoid crystal is 0.05-0.2:1.

Further, the solvent for the suspended organic phase of the carotenoid crystal is a second organic solvent.

Further, the first organic solvent and the second organic solvent are each independently selected from at least one of dichloromethane, tetrahydrofuran, acetone, n-hexane, dimethyl carbonate, ethyl acetate, isopropyl acetate, and methyl tert-butyl ether, preferably dichloromethane.

Further, the material of the spiral coil is stainless steel or copper tube.

Further, the coil heat exchanger further comprises a jacket, which is provided outside the spiral coil and is used for introducing a heating medium.

Further, in the step S2, the mass ratio of the aqueous phase matrix of the colloidal solution to the oil phase matrix of the carotenoid dissolution solution is 1.5-6:1.

Further, the homogenization treatment is carried out under pressure, with a pressure ranging from 20 MPa to 60 MPa, preferably from 35 MPa to 45 MPa.

Further, in the step S2, the mass ratio of the polymer protective colloid, the small molecular carrier, the second antioxidant, and water in the aqueous phase matrix of the colloidal solution is 10-70:0-60:0.5-3:100.

Further, the polymer protective colloid is selected from at least one of modified starch, gelatin, lignosulfonate, sodium caseinate, soy protein, arabic gum or xanthan gum, preferably modified starch or lignosulfonate.

Further, the small molecular carrier is selected from at least one of sucrose, glucose, fructose, trehalose, maltodextrin, resistant dextrin, xanthodextrin, sorbitol or mannitol.

Further, the second antioxidant is selected from at least one of vitamin C, sodium vitamin C, or sodium isovitamin C.

Further, in the step S3, the viscosity number of the carotenoid emulsion is from 100 cp to 2,000 cp.

In the step S4, the spray granulation is carried out in a granulation column containing a starch flow.

Further, the step S4 further comprises a starch separation step after drying, preferably the drying and the starch separation are each independently carried out in a fluidized bed.

Further, the spray granulation comprises spraying the carotenoid emulsion into the granulation column containing a starch flow through a centrifugal sprayer, the air inlet temperature of the granulation column is preferably from 100°C to 130°C, the rotational speed of the centrifugal sprayer is preferably from 8,000 r/min to 15,000 r/min, and the particle size of the starch in the starch flow is preferably less than or equal to 100 mesh.

Further, the weight loss on drying of the carotenoid agent is less than or equal to 8 wt%.

Further, the carotenoid crystal is selected from at least one of β-carotene crystals, canthaxanthin crystals, astaxanthin crystals, lutein crystals, lycopene crystals, apoester crystals, or zeaxanthin crystals.

By applying the technical solution of the present disclosure, the suspended organic dispersion phase of the carotenoid crystal is mixed with a first organic solvent in a spiral coil, and the carotenoid crystal is dissolved in the first organic solvent at 50°C to 70°C, effectively avoiding the drawbacks of isomerization reaction of the carotenoid agent when it is dissolved in traditional preparation methods, the content of all-trans-forms of the carotenoid in the oil phase matrix of the carotenoid dissolution solution obtained is greater than or equal to 90%, and more advantageously stable and highly active carotenoid agent will be obtained subsequently.

### Detailed Description of the Embodiments

It should be noted that the examples and features in the examples in the present disclosure can be combined with each other without conflicting. The present disclosure will be described in detail below in combination with embodiments.

As analyzed in the background art of the present disclosure, it is difficult for the preparation methods of carotenoids in the prior art to overcome the problem of isomerization reactions that occur easily during the dissolution of carotenoids in an organic solvent, resulting in low content of all-trans isomers and low biological activity in carotenoid feed additive products. To solve this problem, the present disclosure provides a preparation method of a carotenoid agent.

In a typical embodiment of the present disclosure, a preparation method of a carotenoid agent is provided, which includes step S1, mixing a suspended organic dispersion phase of a carotenoid crystal with a first organic solvent in a spiral coil of a coil heat exchanger to form a mixed solution, with the carotenoid crystal in the suspended organic dispersion phase of the carotenoid crystal being dissolved in the first organic solvent to obtain an oil phase matrix of the carotenoid dissolution solution, wherein the temperature inside the spiral coil is from 50°C to 70°C.

By applying the technical solution of the present disclosure, the suspended organic dispersion phase of the carotenoid crystal is mixed with a first organic solvent in a spiral coil, and the carotenoid crystal is dissolved in the first organic solvent at 50°C to 70°C, effectively avoiding the drawbacks of isomerization reaction of the carotenoid agent when it is dissolved in traditional preparation methods, the content of all-trans-forms of the carotenoid in the oil phase matrix of the carotenoid dissolution solution obtained is greater than or equal to 90%, and more advantageously stable and highly active carotenoid agent will be obtained subsequently.

The specific types of the above carotenoid crystals are not specifically limited, which include but are not limited to the mixed carotenoid crystals formed from any one or more of β-carotene crystals, canthaxanthin crystals, astaxanthin crystals, lutein crystals, lycopene crystals, apoester crystals, or zeaxanthin crystals.

In order to further shorten the dissolution time and reduce the probability of occurring isomerization reaction of carotenoid, the mass content of the carotenoid crystal in the suspended organic dispersion phase of the carotenoid crystal is preferably from 20% to 40%.

The mass content of the all-trans carotenoids in the above carotenoid crystals is not limited. In order to further improve the content and activity of the all-trans forms in the carotenoid agents subsequently prepared, the mass content of the all-trans forms in the carotenoid crystal is preferably greater than or equal to 95%.

In order to further shorten the dissolution time and reduce the probability of occurring isomerization reaction of carotenoid, the temperature of the suspended organic dispersion phase of the carotenoid crystals before mixing is from 15°C to 30°C, and the temperature of the first organic solvent is from 50°C to 70°C.

In order to further promote the dissolution of carotenoid crystals in the first organic solvent, the mass ratio of the first organic solvent to the carotenoid crystals is preferably 10-20:1.

Typically but non-restrictively, in the present disclosure, the temperature inside the spiral coil is for example 50°C, 52°C, 55°C, 58°C, 60°C, 62°C, 65°C, 68°C, 70°C, or a range of values consisting of any two values; the mass content of the carotenoid crystals in the suspended organic dispersion phase of the carotenoid crystals is for example 20%, 22%, 25%, 28%, 30%, 32%, 35%, 38%, 40%, or a range of values consisting of any two values; the temperature of the suspended organic dispersion phase of the carotenoid crystals, is for example 15°C, 18°C, 20°C, 22°C, 25°C, 28°C, 30°C, or a range of values consisting of any two values; the temperature of the first organic solvent is for example 50°C, 52°C, 55°C, 58°C, 60°C, 62°C, 65°C, 68°C, 70°C, or a range of values consisting of any two values; and the mass ratio of the first organic solvent to the carotenoid crystals is for example 10:1, 12:1, 15:1, 18:1, 20:1, or a range of values consisting of any two values.

In order to further improve the oxidation resistance of the prepared carotenoid agents, it is preferred that the above suspended organic dispersion phase of the carotenoid also includes a first antioxidant. The specific types of the first antioxidant are not limited, which include but are not specifically limited to at least one of dibutylhydroxytoluene (BHT), butylated hydroxyanisole (BHA), tert-butylhydroquinone (TBHQ), vitamin C palmitate, sodium ascorbate, ethoxyquinoline, dl-α tocopherol, especially when the first antioxidant is dl-α tocopherol, its oxidation resistance is better.

The dosage of the above first antioxidant is not specifically limited. In order to reduce the waste of raw materials, the mass ratio of the first antioxidant to the carotenoid crystals is preferably 0.05-0.2:1.

Typically but non-restrictively, the mass ratio of the first antioxidant to the carotenoid crystals is for example 0.05:1, 0.08:1, 0.1:1, 0.12:1, 0.15:1, 0.18:1, 0.2:1, or a range of values consisting of any two values.

The solvent for the above suspended organic phase of the carotenoid crystals is a second organic solvent. The specific types of the first and second solvents are not limited, which include but are not limited to mixed organic solvents formed from any one or more of dichloromethane, tetrahydrofuran, acetone, n-hexane, dimethyl carbonate, ethyl acetate, isopropyl acetate, and methyl tert-butyl ether, especially when the first and second organic solvents are each independently dichloromethane, it is more conducive to improving the mass content of carotenoids in the oil phase matrix of the carotenoid dissolution solution.

In some embodiments of the present disclosure, the preparation method of the above suspended organic dispersion phase of the carotenoid is carried out according to the following steps: mixing the carotenoid crystal, the first antioxidant, and the first organic solvent while stirring in a kettle to form the suspended organic dispersion phase of the carotenoid.

In order to further reduce the isomerization of carotenoids during the dissolution process, the aspect ratio of the above spiral coil is 500-2,000:1, for example 500:1, 800:1, 1,000:1 1,200:1, 1,500:1, 1,800:1, 2,000:1, or a range of values consisting of any two values. Further, the inner diameter of the spiral coil is from 0.5 cm to 2 cm and the length is from 5 m to 20 m; the inner diameter is for example 0.5 cm, 0.8 cm, 1.0 cm, 1.2 cm, 1.8 cm, 2.0 cm, or a range of values consisting of any two values, and the length is for example 5 m, 8 m, 10 m, 12 m, 15 m, 18 m, 20 m, or a range of values consisting of any two values.

As mentioned above, in order to further reduce the isomerization of carotenoids during the dissolution process, the mixed solution formed by mixing the suspended organic dispersion phase of the carotenoid crystal and the first organic solvent in the spiral coil of the coil heat exchanger has a residence time of 40 seconds to 60 seconds in the coil heat exchanger.

In order to avoid corrosion of the above spiral coil by the mixed solution, the material for the above spiral coil is preferably stainless steel or steel tube.

In order to further maintain the stability of the internal temperature of the spiral coil, it is preferred that the coil heat exchanger further comprises a jacket, which is provided on the outer surface of the spiral coil and is used for introducing a heating medium.

In order to prepare acarotenoid agent, the above preparation method further comprises steps S2 to S4 arranged after step S1, among them, step S2 includes mixing the aqueous phase matrix of the colloidal solution with the oil phase matrix of the carotenoid dissolution solution, and performing emulsification and dispersion as well as homogenization treatment successively to obtain a carotenoid nanodispersion, wherein, the aqueous phase matrix of the colloidal solution comprises a polymer protective colloid, a small molecular carrier, a second antioxidant, and water, and the second antioxidant is a water-soluble antioxidant; step S3 includes removing the organic solvent from the carotenoid nanodispersion and concentrating the moisture to obtain a carotenoid emulsion; and step S4 includes subjecting the carotenoid emulsion to spray granulation and drying successively to obtain the carotenoid agent.

In the above steps S2 to S4, the aqueous phase matrix of the colloidal solution and the oil phase matrix of the carotenoid dissolution solution are mixed for performing emulsification, homogenization, desolvation, spray granulation and drying successively. The dispersed particle size D₅₀ of carotenoid active ingredients in the prepared carotenoid preparation is less than or equal to 250 nm, the content of all-trans forms of carotenoid is greater than or equal to 90%, and the content retention rate of 6-month accelerated stability is greater than or equal to 95%, which has excellent activity and stability.

In the above step S2, in order to further improve the emulsification efficiency, the mass ratio of the aqueous phase matrix of the colloidal solution to the oil phase matrix of the carotenoid dissolution solution is preferably 1.5-6:1, such as 1.5:1, 2:1, 3:1, 4:1, 5:1, 6:1, or a range of values consisting of any two values.

In order to further improve the homogenization efficiency, it is preferred to perform the above homogenization under high pressure, with a pressure of 20 MPa to 60 MPa, especially when the pressure is 35 MPa to 45 MPa, the homogenization efficiency is higher.

In order to further promote emulsification efficiency, the mass ratio of the polymer protective colloid, the small molecular carrier, the second antioxidant, and water in the aqueous phase matrix of the colloidal solution is preferably 10-70:0-60:0.5-3:100.

The specific types of the above polymer protective colloids are not specifically limited, which include but are not limited to mixtures formed from any one or more of modified starch, gelatin, lignosulfonate, sodium caseinate, soy protein, arabic gum or xanthan gum. Especially, when the polymer protective colloid is modified starch or lignosulfonate, the aqueous phase matrix of the colloidal solution prepared therefrom is easier to be mixed evenly with the oil phase matrix of the carotenoid dissolution solution.

The specific types of the above small molecular carriers are not specifically limited. Commonly used small molecular carriers in the art can be used, which include but are not limited to mixtures formed from any one or more of sucrose, glucose, fructose, trehalose, maltodextrin, resistant dextrin, xanthodextrin, sorbitol or mannitol.

The specific types of the above second antioxidants are not limited, and any water-soluble antioxidant can be used, which include but are not limited to mixtures formed from any one or more of vitamin C, sodium vitamin C, or sodium isovitamin C.

In order to facilitate spray granulation, in the step S3, the viscosity number of the carotenoid emulsion is preferably from 100 cp to 2,000 cp.

In order to further improve the efficiency of spray granulation, in the step S4, the spray granulation is preferably carried out in the granulation column containing a starch flow, and in order to remove the starch introduced in the granulation process, the step S4 also includes the starch separation step arranged after drying, and the drying and the starch separation are each independently carried out in the fluidized bed.

In some embodiments of the present carotenoid, the spray granulation is carried out according to the following steps: spraying the carotenoid emulsion into the granulation column containing a starch flow through a centrifugal sprayer, wherein the air inlet temperature of the granulation column is from 100°C to 130°C so as to further remove moisture. In order to further improve the efficiency of spray granulation, the rotational speed of the centrifugal spray is preferably from 8,000 r/min to 15,000 r/min.

In order to further improve the regularity of the carotenoid agents, the particle size of starch in the starch flow is preferably less than or equal to 100 mesh.

During the above drying process, the weight loss on drying of the carotenoid preparation is preferably controlled to be less than or equal to 8 wt%.

The beneficial effects of the present carotenoid will be further explained below in combination with examples and comparative examples.

### Example 1

This example provided a canthaxanthin particulate product, which was prepared according to the following steps:
(1) mixing 22 kg of canthaxanthin crystal (with a content of all-trans forms of 98.8 wt%), 4 kg of dl-α-tocopherol and 29 kg of dichloromethane evenly while stirring in a kettle, cooling to 15°C to obtain 40 wt% suspended organic dispersion phase of canthaxanthin; heating 220 kg of dichloromethane to 50°C in another kettle for later use; dissolving 80 kg of lignosulfonate, 54 kg of maltodextrin, and 300 kg of water in the kettle at elevated temperature and then cooling to 35°C to obtain the aqueous phase matrix of the colloidal solution.
(2) delivering the above suspended organic dispersion phase of canthaxanthin at a flow rate of 2.6 kg/min, dichloromethane stock solution of 50°C at a flow rate of 10.5 kg/min into the spiral coil of a copper coil heater with an inner diameter of 1 cm, a length of 5 m and a jacket water bath temperature set at 50°C through a pump and mixing for a residence time of 40 seconds before flowing out of the outlet of the copper coil heater, with the outlet temperature being monitored to be 43°C to 47°C, so as to obtain the oil phase matrix of the canthaxanthin dissolution solution.
(3) instantaneously mixing and emulsifying the oil phase matrix of the canthaxanthin dissolution solution and the aqueous phase matrix of the colloidal solution through a pipeline emulsion pump to obtain a crude emulsion. Then, the crude emulsion was homogenized and nano-dispersed at a condition of 60 MPa by high-pressure homogenization equipment. After the homogenization was completed, the organic solvent dichloromethane was removed by desolventizing under reduced pressure. After desolvation, the emulsion was dehydrated and concentrated through a film evaporator to give canthaxanthin emulsion with a viscosity number of 100 cp (with a mass of solution being about 290 kg; and a concentration of solid content being about 55 wt%). The canthaxanthin emulsion was granulated by centrifugal spray ring spraying in a granulation column containing a starch flow with an air inlet temperature of 100 °C and an atomizer rotational speed of 8,000 r/min, and a layer of dry starch was adsorbed on the surface of the fog droplets for molding and preliminary drying, and the starch containing canthaxanthin particles were dried again in the subsequent fluidized bed to achieve starch separation. Finally, sieving was carried out, and the canthaxanthin microcapsule products with a particle size of 20 to 120 mesh were collected to obtain 190 kg of the canthaxanthin particulate product.

The product was subjected to quality testing, and the results showed that the mass content of canthaxanthin in the canthaxanthin particulate product was 10.8%, the mass content of all-trans isomers was 91.5%, the dispersed particle size D₅₀ of the canthaxanthin active ingredient was 195 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the canthaxanthin particulate product as detected by HPLC was 95.7%.

### Example 2

This example provided an astaxanthin particulate product, which was prepared according to the following steps:
(1) mixing 22 kg of astaxanthin crystal (with a content of all-trans forms of 98.8 wt%), 2 kg of BHA, 2 kg of ethoxyquinoline and 48 kg of dichloromethane evenly at room temperature while stirring in a kettle to obtain 30 wt% suspended organic dispersion phase of astaxanthin; mixing 100 kg of tetrahydrofuran, 50 kg of acetone, 50 kg of dimethyl carbonate, and 52 kg of ethyl acetate in another kettle to obtain the first organic solvent, which was heated to 50 °C for later use; dissolving 40 kg of gelatin, 40 kg of xanthodextrin, 40 kg of glucose, 11 kg of sorbitol, 3 kg of vitamin C palmitate, and 300 kg of water in the kettle at elevated temperature and then cooling to 35°C to obtain the aqueous phase matrix of the colloidal solution.
(2) delivering the above suspended organic dispersion phase of astaxanthin at a flow rate of 3.1 kg/min, the first organic solvent of 50°C at a flow rate of 10.5 kg/min into the spiral coil of a copper coil heater with an inner diameter of 1 cm, a length of 5 m and a jacket water bath temperature set at 50°C through a pump and mixing for a residence time of 48 seconds before flowing out of the outlet of the copper coil heater, with the outlet temperature being monitored to be 43°C to 47°C, so as to obtain the oil phase matrix of the astaxanthin dissolution solution.
(3) instantaneously mixing and emulsifying the oil phase matrix of the astaxanthin dissolution solution and the aqueous phase matrix of the colloidal solution through a pipeline emulsion pump to obtain a crude emulsion. Then, the crude emulsion was homogenized and nano-dispersed at a condition of 20 MPa by high-pressure homogenization equipment. After the homogenization was completed, the organic solvent was removed by desolventizing under reduced pressure. After desolvation, the emulsion was dehydrated and concentrated through a film evaporator to give astaxanthin emulsion with a viscosity number of 300 cp (with a mass of solution being about 290 kg; and a concentration of solid content being about 55 wt%). The astaxanthin emulsion was granulated by centrifugal spray ring spraying in a granulation column containing a starch flow with an air inlet temperature of 120°C and an atomizer rotational speed of 10,000 r/min, and a layer of dry starch was adsorbed on the surface of the fog droplets for molding and preliminary drying, and the starch containing astaxanthin particles were dried again in the subsequent fluidized bed to achieve starch separation. Finally, sieving was carried out, and the astaxanthin microcapsule products with a particle size of 20 to 120 mesh were collected to obtain 192 kg of the astaxanthin particulate product.

The product was subjected to quality testing, and the results showed that the content of astaxanthin in the astaxanthin particulate product was 10.6%, the mass content of all-trans isomers was up to 91.0%, the dispersed particle size D₅₀ of the astaxanthin active ingredient was 200 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 95.9%.

### Example 3

This example provided an apoester particulate product, which was prepared according to the following steps:
(1) mixing 22 kg of apoester crystal (with a content of all-trans forms of 97.8 wt%), 2 kg of BHT, 2 kg of TBHQ and 48 kg of dichloromethane evenly at room temperature while stirring in a kettle to obtain 30 wt% suspended organic dispersion phase of apoester; mixing 100 kg of dichloromethane, 50 kg of n-hexane, 50 kg of isopropyl acetate, and 52 kg of methyl tert-butyl ether in another kettle to obtain the first organic solvent, which was heated to 50°C for later use; dissolving 30 kg of sodium caseinate, 10 kg of soy protein, 70 kg of resistant dextrin, 24 kg of mannitol, and 300 kg of water in the kettle at elevated temperature and then cooling to 35°C to obtain the aqueous phase matrix of the colloidal solution.
(2) delivering the above suspended organic dispersion phase of apoester at a flow rate of 3.1 kg/min, the first organic solvent of 50°C at a flow rate of 10.5 kg/min into the spiral coil of a copper coil heater with an inner diameter of 1 cm, a length of 5 m and a jacket water bath temperature set at 50°C through a pump and mixing for a residence time of 50 seconds before flowing out of the outlet of the copper coil heater, with the outlet temperature being monitored to be 43°C to 47°C, so as to obtain the oil phase matrix of the apoester dissolution solution.
(3) instantaneously mixing and emulsifying the oil phase matrix of the apoester dissolution solution and the aqueous phase matrix of the colloidal solution through a pipeline emulsion pump to obtain a crude emulsion. Then, the crude emulsion was homogenized and nano-dispersed at a condition of 40 MPa by high-pressure homogenization equipment. After the homogenization was completed, the organic solvent was removed by desolventizing under reduced pressure. After desolvation, the emulsion was dehydrated and concentrated through a film evaporator to give apoester emulsion with a viscosity number of 500 cp (with a mass of solution being about 290 kg; and a concentration of solid content being about 55 wt%). The apoester emulsion was granulated by centrifugal spray ring spraying in a granulation column containing a starch flow with an air inlet temperature of 120°C and an atomizer rotational speed of 13,000 r/min, and a layer of dry starch was adsorbed on the surface of the fog droplets for molding and preliminary drying, and the starch containing apoester particles were dried again in the subsequent fluidized bed to achieve starch separation. Finally, sieving was carried out, and the apoester microcapsule products with a particle size of 20 to 120 mesh were collected to obtain 185 kg of the apoester particulate product.

The product was subjected to quality testing, and the results showed that the mass content of apoester in the apoester particulate product was 10.8%, the mass content of all-trans isomers was up to 91.5%, the dispersed particle size D₅₀ of the apoester active ingredient was 195 nm. Upon 6 months of accelerated stability testing (experimental conditions: 40°C± 2.0°C, 75% R.H ± 5% R.H), the study results showed that the content retention rate (mass content of accelerated product /initial mass content of product) of the product as detected by HPLC was 95.8%.

### Example 4

This example provided a β-carotene particulate product, which was prepared according to the following steps:
(1) mixing 23 kg of β-carotene crystal (with a content of all-trans forms of 97.8 wt%), 4 kg of dl-α-tocopherol and 48 kg of dichloromethane evenly at room temperature while stirring in a kettle, to obtain 30 wt% suspended organic dispersion phase of β-carotene; heating 363 kg of dichloromethane to 68°C in another kettle for later use; dissolving 110 kg of sodium starch octenyl succinate, 33 kg of sucrose, and 300 kg of water in the kettle at elevated temperature and then cooling to 35°C to obtain the aqueous phase matrix of the colloidal solution.
(2) delivering the above suspended organic dispersion phase of β-carotene at a flow rate of 3.0 kg/min, dichloromethane stock solution of 68°C at a flow rate of 14.5 kg/min into the spiral coil of a copper coil heater with an inner diameter of 1 cm, a length of 5 m and a jacket water bath temperature set at 65°C through a pump and mixing for a residence time of 60 seconds before flowing out of the outlet of the copper coil heater, with the outlet temperature being monitored to be 58°C to 63°C, so as to obtain the oil phase matrix of the β-carotene dissolution solution.
(3) instantaneously mixing and emulsifying the oil phase matrix of the β-carotene dissolution solution and the aqueous phase matrix of the colloidal solution through a pipeline emulsion pump to obtain a crude emulsion. Then, the crude emulsion was homogenized and nano-dispersed at a condition of 50 MPa by high-pressure homogenization equipment. After the homogenization was completed, the organic solvent dichloromethane was removed by desolventizing under reduced pressure. After desolvation, the emulsion was dehydrated and concentrated through a film evaporator to give β-carotene emulsion with a viscosity number of 800 cp (with a mass of solution being about 310 kg; and a concentration of solid content being about 55 wt%). The β-carotene emulsion was granulated by centrifugal spray ring spraying in a granulation column containing a starch flow with an air inlet temperature of 130°C and an atomizer rotational speed of 15,000 r/min, and a layer of dry starch was adsorbed on the surface of the fog droplets for molding and preliminary drying, and the starch containing β-carotene particles were dried again in the subsequent fluidized bed to achieve starch separation. Finally, sieving was carried out, and the β-carotene microcapsule products with a particle size of 20 to 120 mesh were collected to obtain 180 kg of the β-carotene particulate product.

The product was subjected to quality testing, and the results showed that the mass content of β-carotene in the β-carotene particulate product was 11.2%, the mass content of all-trans isomers was up to 95.2%, the dispersed particle size D₅₀ of the β-carotene active ingredient was 215 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 99.0%.

### Example 5

This example provided a lycopene particulate product, which was prepared according to the following steps:
(1) mixing 23 kg of lycopene crystal (with a content of all-trans forms of 98.5 wt%), 4 kg of dl-α-tocopherol and 48 kg of dichloromethane evenly at room temperature while stirring in a kettle, to obtain 30 wt% suspended organic dispersion phase of lycopene; heating 363 kg of dichloromethane to 70°C in another kettle for later use; dissolving 113 kg of sodium starch octenyl succinate, 20 kg of fructose, 13 kg of trehalose and 300 kg of water in the kettle at elevated temperature and then cooling to 35°C to obtain the aqueous phase matrix of the colloidal solution.
(2) delivering the above suspended organic dispersion phase of lycopene at a flow rate of 3.0 kg/min, dichloromethane stock solution of 70°C at a flow rate of 14.5 kg/min into the spiral coil of a copper coil heater with an inner diameter of 1 cm, a length of 5 m and a jacket water bath temperature set at 65°C through a pump and mixing for a residence time of 55 seconds before flowing out of the outlet of the copper coil heater, with the outlet temperature being monitored to be 58°C to 63°C, so as to obtain the oil phase matrix of the lycopene dissolution solution.
(3) instantaneously mixing and emulsifying the oil phase matrix of the lycopene dissolution solution and the aqueous phase matrix of the colloidal solution through a pipeline emulsion pump to obtain a crude emulsion. Then, the crude emulsion was homogenized and nano-dispersed at a condition of 45 MPa by high-pressure homogenization equipment. After the homogenization was completed, the organic solvent dichloromethane was removed by desolventizing under reduced pressure. After desolvation, the emulsion was dehydrated and concentrated through a film evaporator to give lycopene emulsion with a viscosity number of 2,000 cp (with a mass of solution being about 310 kg; and a concentration of solid content being about 55 wt%). The lycopene emulsion was granulated by centrifugal spray ring spraying in a granulation column containing a starch flow with an air inlet temperature of 120°C and an atomizer rotational speed of 12,000 r/min, and a layer of dry starch was adsorbed on the surface of the fog droplets for molding and preliminary drying, and the starch containing lycopene particles were dried again in the subsequent fluidized bed to achieve starch separation. Finally, sieving was carried out, and the lycopene microcapsule products with a particle size of 20 to 120 mesh were collected to obtain 185 kg of the lycopene particulate product.

The product was subjected to quality testing, and the results showed that the mass content of lycopene in the lycopene particulate product was 11.3%, the mass content of all-trans isomers was up to 95.6%, the dispersed particle size D₅₀ of the lycopene active ingredient was 215 nm. Upon 6 months of accelerated stability testing (experimental conditions: 40°C± 2.0°C, 75% R.H ± 5% R.H), the study results showed that the content retention rate (mass content of accelerated product /initial mass content of product) of the product as detected by HPLC was 98.4%.

### Example 6

This example provided a lutein particulate product, which was prepared according to the following steps:
(1) mixing 14 kg of lutein crystal (with a content of 80.5 wt%), 14 kg of zeaxanthin crystal (with a content of 80.3 wt%), 3 kg of dl-α-tocopherol and 30 kg of dichloromethane evenly at room temperature while stirring in a kettle, to obtain 30% suspended organic dispersion phase of lutein; heating 220 kg of dichloromethane to 70°C in another kettle for later use; dissolving 110 kg of sodium starch octenyl succinate, 26 kg of sucrose, 3 kg of sodium ascorbate and 300 kg of water in the kettle at elevated temperature and then cooling to 35°C to obtain the aqueous phase matrix of the colloidal solution.
(2) delivering the above suspended organic dispersion phase of lutein and zeaxanthin at a flow rate of 3.0 kg/min, dichloromethane stock solution of 70°C at a flow rate of 14.0 kg/min into the spiral coil of a copper coil heater with an inner diameter of 1 cm, a length of 5 m and a jacket water bath temperature set at 65°C through a pump and mixing for a residence time of 55 seconds before flowing out of the outlet of the copper coil heater, with the outlet temperature being monitored to be 60°C to 65°C, so as to obtain the oil phase matrix of the lutein and zeaxanthin dissolution solution.
(3) instantaneously mixing and emulsifying the oil phase matrix of the lutein and zeaxanthin dissolution solution and the aqueous phase matrix of the colloidal solution through a pipeline emulsion pump to obtain a crude emulsion. Then, the crude emulsion was homogenized and nano-dispersed at a condition of 40 MPa by high-pressure homogenization equipment. After the homogenization was completed, the organic solvent dichloromethane was removed by desolventizing under reduced pressure. After desolvation, the emulsion was dehydrated and concentrated through a film evaporator to give lutein and zeaxanthin emulsion with a viscosity number of 1,500 cp (with a mass of solution being about 310 kg; and a concentration of solid content being about 55 wt%). The lutein and zeaxanthin emulsion was granulated by centrifugal spray ring spraying in a granulation column containing a starch flow with an air inlet temperature of 120°C and an atomizer rotational speed of 12,000 r/min, and a layer of dry starch was adsorbed on the surface of the fog droplets for molding and preliminary drying, and the starch containing lutein particles were dried again in the subsequent fluidized bed to achieve starch separation. Finally, sieving was carried out, and the lutein microcapsule products with a particle size of 20 to 120 mesh were collected to obtain 185 kg of the lutein and zeaxanthin particulate product.

The product was subjected to quality testing, and the results showed that the mass content of lutein in the lutein and zeaxanthin particulate product was 5.5%, the mass content of zeaxanthin was 5.8%, the mass content of all-trans isomers was up to 92.5%, the dispersed particle size D₅₀ of the lutein and zeaxanthin active ingredients was 210 nm. Upon 6 months of accelerated stability testing (experimental conditions: 40°C± 2.0°C, 75% R.H ± 5% R.H), the study results showed that the content retention rate of lutein as detected by HPLC was 95.6%, and the content retention rate of zeaxanthin was 95.1% (mass content of accelerated product /initial mass content of product).

### Example 7

The difference between this example and example 4 lied in that in step (1), the suspended organic dispersion phase of β-carotene was heated up to 30°C for later use.

The product was subjected to quality testing, and the results showed that the mass content of β-carotene in the β-carotene particulate product was 11.1%, the mass content of all-trans isomers was 95.1%, the dispersed particle size D₅₀ of the β-carotene active ingredient was 215 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 99.0%.

### Example 8

The difference between this example and example 4 lied in that in step (1), the suspended organic dispersion phase of β-carotene was cooled down to 15°C for later use.

The product was subjected to quality testing, and the results showed that the mass content of β-carotene in the β-carotene particulate product was 11.2%, the mass content of all-trans isomers was 95.3%, the dispersed particle size D₅₀ of the β-carotene active ingredient was 215 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 98.8%.

### Example 9

The difference between this example and example 4 lied in that the inner diameter of the spiral coil in the copper coil heater was 0.5cm, the length was 5 m, and in step (2), the residence time of the mixed solution in the spiral coil was made to be 60 seconds by regulating the flow rate of the suspended organic dispersion phase of β-carotene and the flow rate of the dichloromethane stock solution.

The product was subjected to quality testing, and the results showed that the mass content of β-carotene in the β-carotene particulate product was 11.0%, the mass content of all-trans isomers was 95.0%, the dispersed particle size D₅₀ of the β-carotene active ingredient was 215 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 98.9%.

### Example 4410

The difference between this example and example 4 lied in that the inner diameter of the spiral coil in the copper coil heater was 2cm, the length was 20m, and in step (2), the residence time of the mixed solution in the spiral coil was made to be 60 seconds by regulating the flow rate of the suspended organic dispersion phase of β-carotene and the flow rate of the dichloromethane stock solution.

The product was subjected to quality testing, and the results showed that the mass content of β-carotene in the β-carotene particulate product was 11.2%, the mass content of all-trans isomers was 95.2%, the dispersed particle size D₅₀ of the β-carotene active ingredient was 215 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 99.0%.

### Comparative example 1

This comparative example provided a canthaxanthin particulate product, which was prepared according to the following steps:
(1) stirring 22 kg of canthaxanthin crystal (with a content of all-trans forms of 98.8 wt%), 4 kg of dl-α-tocopherol and 249 kg of dichloromethane in a kettle and heating up to 50°C for later use; dissolving 80 kg of lignosulfonate, 54 kg of maltodextrin, and 300 kg of water in the kettle at elevated temperature and then cooling to 35°C to obtain the aqueous phase matrix of the colloidal solution.
(2) instantaneously mixing and emulsifying the above oil phase matrix of the canthaxanthin dissolution solution at a flow rate of 13.1 kg/min and the aqueous phase matrix of the colloidal solution through a pipeline emulsion pump to obtain a crude emulsion, wherein during this process, it took 55 minutes from the oil phase to be heated up at the beginning until it was dissolved to the end of emulsification. Then, the crude emulsion was homogenized and nano-dispersed at a condition of 60 MPa by high-pressure homogenization equipment. After the homogenization was completed, the organic solvent dichloromethane was removed by desolventizing under reduced pressure. After desolvation, the emulsion was dehydrated and concentrated through a film evaporator to give canthaxanthin emulsion with a viscosity number of 100 cp (with a mass of solution being about 290 kg; and a concentration of solid content being about 55 wt%). The canthaxanthin emulsion was granulated by centrifugal spray ring spraying in a granulation column containing a starch flow with an air inlet temperature of 100 °C and an atomizer rotational speed of 8,000 r/min, and a layer of dry starch was adsorbed on the surface of the fog droplets for molding and preliminary drying, and the starch containing canthaxanthin particles were dried again in the subsequent fluidized bed to achieve starch separation. Finally, sieving was carried out, and the canthaxanthin microcapsule products with a particle size of 20 to 120 mesh were collected to obtain 191kg of the canthaxanthin particulate product.

The product was subjected to quality testing, and the results showed that the content of canthaxanthin in the canthaxanthin particulate product was 10.3%, the mass content of all-trans isomers was 75.5%, the dispersed particle size D₅₀ of the canthaxanthin active ingredient was 205 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 95.7%.

### Comparative example 2

This comparative example provided a canthaxanthin particulate product, which was prepared according to the following steps:
(1) stirring 22 kg of canthaxanthin crystal (with a content of all-trans forms of 98.8 wt%), 4 kg of dl-α-tocopherol and 249 kg of dichloromethane in a kettle and heating up to 50°C to prepare the oil phase matrix of the canthaxanthin dissolution solution for later use; dissolving 80 kg of lignosulfonate, 54 kg of maltodextrin, and 300 kg of water in the kettle at elevated temperature and then cooling to 35°C to obtain the aqueous phase matrix of the colloidal solution.
(2) instantaneously mixing and emulsifying the above oil phase matrix of the canthaxanthin dissolution solution at a flow rate of 13.1 kg/min and the aqueous phase matrix of the colloidal solution through a pipeline emulsion pump to obtain a crude emulsion, wherein during this process, it took 55 minutes from the oil phase to be heated up at the beginning until it was dissolved to the end of emulsification. Then, the organic solvent dichloromethane was removed from the crude emulsion by desolventizing under reduced pressure. After desolvation, the emulsion was dehydrated and concentrated through a film evaporator to give canthaxanthin emulsion with a viscosity number of 100 cp (with a mass of solution being about 290 kg; and a concentration of solid content being about 55 wt%). The canthaxanthin emulsion was granulated by centrifugal spray ring spraying in a granulation column containing a starch flow with an air inlet temperature of 100 °C and an atomizer rotational speed of 8,000 r/min, and a layer of dry starch was adsorbed on the surface of the fog droplets for molding and preliminary drying, and the starch containing canthaxanthin particles were dried again in the subsequent fluidized bed to achieve starch separation. Finally, sieving was carried out, and the canthaxanthin microcapsule products with a particle size of 20 to 120 mesh were collected to obtain 183kg of the canthaxanthin particulate product.

The product was subjected to quality testing, and the results showed that the mass content of canthaxanthin in the canthaxanthin particulate product was 10.2%, the mass content of all-trans isomers was 75.6%, the dispersed particle size D₅₀ of the canthaxanthin active ingredient was 2,350 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 88.7%.

### Comparative example 3

This comparative example provided a β-carotene particulate product, which was prepared according to the following steps:
(1) stirring 23 kg of β-carotene crystal (with a content of all-trans forms of 97.8 wt%), 4 kg of dl-α-tocopherol and 411 kg of dichloromethane at room temperature in a kettle and then heating up to 60°C to prepare the oil phase matrix of the β-carotene dissolution solution for later use; dissolving 110 kg of sodium starch octenyl succinate, 33 kg of sucrose, and 300 kg of water in the kettle at elevated temperature and then cooling to 35°C to obtain the aqueous phase matrix of the colloidal solution.
(2) instantaneously mixing and emulsifying the above oil phase matrix of the β-carotene dissolution solution at a flow rate of 17.5 kg/min and the aqueous phase matrix of the colloidal solution through a pipeline emulsion pump to obtain a crude emulsion, wherein during this process, it took 65 minutes from the oil phase to be heated up at the beginning until it was dissolved to the end of emulsification. Then, the crude emulsion was homogenized and nano-dispersed at a condition of 50 MPa by high-pressure homogenization equipment. After the homogenization was completed, the organic solvent dichloromethane was removed by desolventizing under reduced pressure. After desolvation, the emulsion was dehydrated and concentrated through a film evaporator to give β-carotene emulsion with a viscosity number of 800 cp (with a mass of solution being about 310 kg; and a concentration of solid content being about 55 wt%). The β-carotene emulsion was granulated by centrifugal spray ring spraying in a granulation column containing a starch flow with an air inlet temperature of 130°C and an atomizer rotational speed of 15,000 r/min, and a layer of dry starch was adsorbed on the surface of the fog droplets for molding and preliminary drying, and the starch containing β-carotene particles were dried again in the subsequent fluidized bed to achieve starch separation. Finally, sieving was carried out, and the β-carotene microcapsule products with a particle size of 20 to 120 mesh were collected to obtain 175 kg of the β-carotene particulate product.

The product was subjected to quality testing, and the results showed that the mass content of β-carotene in the β-carotene particulate product was 10.6%, the mass content of all-trans isomers was 85.2%, the dispersed particle size D₅₀ of the β-carotene active ingredient was 235 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 95.2%.

### Comparative example 4

This comparative example provided a β-carotene particulate product, which was prepared according to the following steps:
(1) stirring 23 kg of β-carotene crystal (with a content of all-trans forms of 97.8 wt%), 4 kg of dl-α-tocopherol and 411 kg of dichloromethane at room temperature in a kettle and then heating up to 60°C to prepare the oil phase matrix of the β-carotene dissolution solution for later use; dissolving 110 kg of sodium starch octenyl succinate, 33 kg of sucrose, and 300 kg of water in the kettle at elevated temperature and then cooling to 35°C to obtain the aqueous phase matrix of the colloidal solution.
(2) instantaneously mixing and emulsifying the above oil phase matrix of the β-carotene dissolution solution at a flow rate of 17.5 kg/min and the aqueous phase matrix of the colloidal solution through a pipeline emulsion pump to obtain a crude emulsion, wherein during this process, it took 65 minutes from the oil phase to be heated up at the beginning until it was dissolved to the end of emulsification. Then, the crude emulsion was homogenized and nano-dispersed at a condition of 10 MPa by high-pressure homogenization equipment. After the homogenization was completed, the organic solvent dichloromethane was removed by desolventizing under reduced pressure. After desolvation, the emulsion was dehydrated and concentrated through a film evaporator to give β-carotene emulsion with a viscosity number of 800 cp (with a mass of solution being about 310 kg; and a concentration of solid content being about 55 wt%). The β-carotene emulsion was granulated by centrifugal spray ring spraying in a granulation column containing a starch flow with an air inlet temperature of 130°C and an atomizer rotational speed of 15,000 r/min, and a layer of dry starch was adsorbed on the surface of the fog droplets for molding and preliminary drying, and the starch containing β-carotene particles were dried again in the subsequent fluidized bed to achieve starch separation. Finally, sieving was carried out, and the β-carotene microcapsule products with a particle size of 20 to 120 mesh were collected to obtain 178 kg of the β-carotene particulate product.

The product was subjected to quality testing, and the results showed that the mass content of β-carotene in the β-carotene particulate product was 10.4%, the mass content of all-trans isomers was 85.3%, the dispersed particle size D50 of the β-carotene active ingredient was 850 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 86.5%.

### Comparative example 5

The difference between this comparative example and example 4 lied in that in step (2), the temperature of the jacket water bath of the copper coil heater was set to 45°C.

The product was subjected to quality testing, and the results showed that the mass content of β-carotene in the β-carotene particulate product was 11.1%, the mass content of all-trans isomers was 95.4%, the dispersed particle size D50 of the β-carotene active ingredient was 720 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 82.7%.

### Comparative example 6

The difference between this comparative example and example 4 lied in that in step (2), the temperature of the jacket water bath of the copper coil heater was set to 80°C.

The product was subjected to quality testing, and the results showed that the mass content of β-carotene in the β-carotene particulate product was 10.5%, the mass content of all-trans isomers was 89.2%, the dispersed particle size D50 of the β-carotene active ingredient was 220 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 93.1%.

From the above description, it can be seen that the above examples of the present disclosure have achieved the following technical effects: by mixing the suspended organic dispersion phase of the carotenoid crystal with a first organic solvent in a spiral coil, and dissolving the carotenoid crystal in the first organic solvent at 50°C to 70°C, the drawbacks of isomerization reaction of the carotenoid agent when it is dissolved in traditional preparation methods can be effectively avoided, and the content of all-trans-forms of the carotenoid in the oil phase matrix of the carotenoid dissolution solution obtained is greater than or equal to 90%, which is more beneficial to obtain stable and highly active carotenoid agents subsequently.

### Comparative example 7

The difference between this example and example 4 lied in that in step (1), the suspended organic dispersion phase of β-carotene was cooled down to 5°C for later use.

The product was subjected to quality testing, and the results showed that the mass content of β-carotene in the β-carotene particulate product was 11.1%, the mass content of all-trans isomers was 95.6%, the dispersed particle size D₅₀ of the β-carotene active ingredient was 320 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 89.4%.

### Comparative example 8

The difference between this example and example 4 lied in that in step (1), the suspended organic dispersion phase of β-carotene was heated up to 50°C for later use.

The product was subjected to quality testing, and the results showed that the mass content of β-carotene in the β-carotene particulate product was 10.5%, the mass content of all-trans isomers was 90.7%, the dispersed particle size D₅₀ of the β-carotene active ingredient was 210 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 95.5%.

### Comparative example 9

The difference between this example and example 4 lied in that in step (1), dichloromethane was at room temperature for later use.

The product was subjected to quality testing, and the results showed that the mass content of β-carotene in the β-carotene particulate product was 11.3%, the mass content of all-trans isomers was 95.3%, the dispersed particle size D₅₀ of the β-carotene active ingredient was 5,650 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 84.7%.

### Comparative example 10

The difference between this example and example 4 lied in that in step (1), dichloromethane was heated up to 80°C for later use.

The product was subjected to quality testing, and the results showed that the mass content of β-carotene in the β-carotene particulate product was 10.1%, the mass content of all-trans isomers was 85.2%, the dispersed particle size D₅₀ of the β-carotene active ingredient was 210 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 93.6%.

### Comparative example 11

The difference between this example and example 4 lied in that in step (2), the residence time of the mixed solution in the spiral coil was made to be 90 seconds by regulating the flow rate of the suspended organic dispersion phase of β-carotene and the flow rate of the dichloromethane stock solution.

The product was subjected to quality testing, and the results showed that the mass content of β-carotene in the β-carotene particulate product was 10.3%, the mass content of all-trans isomers was 87.3%, the dispersed particle size D₅₀ of the β-carotene active ingredient was 225 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 93.5%.

### Comparative example 12

The difference between this example and example 4 lied in that in step (2), the residence time of the mixed solution in the spiral coil was made to be 20 seconds by regulating the flow rate of the suspended organic dispersion phase of β-carotene and the flow rate of the dichloromethane stock solution.

The product was subjected to quality testing, and the results showed that the mass content of β-carotene in the β-carotene particulate product was 11.2%, the mass content of all-trans isomers was 95.3%, the dispersed particle size D₅₀ of the β-carotene active ingredient was 950 nm. Upon 6 months of accelerated stability testing (experimental conditions: 40 °C± 2.0 °C, 75% R.H ± 5% R.H), the study results showed that the content retention rate (mass content of accelerated product /initial mass content of product) of the product as detected by HPLC was 83.6%.

## Claims

1. A preparation method of carotenoid agent, wherein, the preparation method comprises:
step S1, mixing a suspended organic dispersion phase of a carotenoid crystal with a first organic solvent in a spiral coil of a coil heat exchanger to form a mixed solution, with the carotenoid crystal in the suspended organic phase of the carotenoid crystal being dissolved in the first organic solvent to obtain an oil phase matrix of the carotenoid dissolution solution, wherein the temperature inside the spiral coil is from 50°C to 70°C;
step S2, mixing the aqueous phase matrix of the colloidal solution with the oil phase matrix of the carotenoid dissolution solution, and performing emulsification and dispersion as well as homogenization treatment successively to obtain a carotenoid nanodispersion, wherein, the aqueous phase matrix of the colloidal solution comprises a polymer protective colloid, a small molecular carrier, a second antioxidant, and water, and the second antioxidant is a water-soluble antioxidant;
step S3, removing the organic solvent from the carotenoid nanodispersion and concentrating the moisture to obtain a carotenoid emulsion; and
step S4, subjecting the carotenoid emulsion to spray granulation and drying successively to obtain the carotenoid agent;
the temperature of the suspended organic dispersion phase of the carotenoid crystal is from 15°C to 30°C, and the temperature of the first organic solvent is from 50 to 70°C;
the residence time of the mixed solution in the coil heat exchanger is from 40 seconds to 60 seconds;
the aspect ratio of the spiral coil is 500-2,000:1; the inner diameter of the spiral coil is from 0.5 cm to 2 cm and the length is from 5 m to 20 m.

2. The preparation method according to claim 1, wherein, the mass content of the carotenoid crystal in the suspended organic dispersion phase of the carotenoid crystal is from 20% to 40%;
and/or, the mass content of the all-trans forms in the carotenoid crystal is greater than or equal to 95%;
and/or, the mass ratio of the first organic solvent to the carotenoid crystal is 7.5-20:1.

3. The preparation method according to claim 1, wherein, the suspended organic dispersion phase of the carotenoid crystal further comprises a first antioxidant, which is selected from at least one of dibutylhydroxytoluene (BHT), butylated hydroxyanisole (BHA), tert-butylhydroquinone (TBHQ), vitamin C palmitate, sodium ascorbate, ethoxyquinoline, dl-α tocopherol.

4. The preparation method according to claim 3, wherein, the mass ratio of the first antioxidant to the carotenoid crystal is 0.05-0.2:1.

5. The preparation method according to claim 1, wherein, the solvent for the suspended organic phase of the carotenoid crystal is a second organic solvent.

6. The preparation method according to claim 5, wherein, the first organic solvent and the second organic solvent are each independently selected from at least one of dichloromethane, tetrahydrofuran, acetone, n-hexane, dimethyl carbonate, ethyl acetate, isopropyl acetate, and methyl tert-butyl ether, preferably dichloromethane.

7. The preparation method according to claim 1, wherein, the material of the spiral coil is stainless steel or copper tube; and/or, the coil heat exchanger further comprises a jacket, which is provided outside the spiral coil and is used for introducing a heating medium.

8. The preparation method according to claim 1, wherein, in the step S2, the mass ratio of the aqueous phase matrix of the colloidal solution to the oil phase matrix of the carotenoid dissolution solution is 1.5-6: 1;
and/or, the homogenization treatment is carried out under pressure, with a pressure ranging from 20 MPa to 60 MPa, preferably from 35 MPa to 45 MPa.

9. The preparation method according to claim 1, wherein, in the step S2, the mass ratio of the polymer protective colloid, the small molecular carrier, the second antioxidant, and water in the aqueous phase matrix of the colloidal solution is 10-70:0-60:0.5-3:100;
and/or the polymer protective colloid is selected from at least one of modified starch, gelatin, lignosulfonate, sodium caseinate, soy protein, arabic gum or xanthan gum, preferably modified starch or lignosulfonate;
and/or, the small molecular carrier is selected from at least one of sucrose, glucose, fructose, trehalose, maltodextrin, resistant dextrin, xanthodextrin, sorbitol or mannitol; and/or, the second antioxidant is selected from at least one of vitamin C, sodium vitamin C, or sodium isovitamin C.

10. The preparation method according to claim 1, wherein, in the step S3, the viscosity number of the carotenoid emulsion is from 100 cp to 2,000 cp.

11. The preparation method according to any one of claims 1 to 10, wherein, in the step S4, the spray granulation is carried out in a granulation column containing a starch flow;
and/or, the step S4 further comprises a starch separation step after drying, preferably the drying and the starch separation are each independently carried out in a fluidized bed;
and/or, the spray granulation comprises spraying the carotenoid emulsion into the granulation column containing a starch flow through a centrifugal sprayer, the air inlet temperature of the granulation column is preferably from 100°C to 130°C, the rotational speed of the centrifugal sprayer is preferably from 8,000 r/min to 15,000 r/min;
and/or, the weight loss on drying of the carotenoid agent is less than or equal to 8 wt%.

12. The preparation method according to any one of claims 1 to 11, wherein, the carotenoid crystal is selected from at least one of β-carotene crystals, canthaxanthin crystals, astaxanthin crystals, lutein crystals, lycopene crystals, apoester crystals, or zeaxanthin crystals.

## Patentansprüche

1. Verfahren zur Herstellung eines Carotinoidmittels, wobei das Herstellungsverfahren umfasst:
Schritt S1: Mischen einer suspendierten organischen Dispersionsphase eines Carotinoidkristalls mit einem ersten organischen Lösungsmittel in einer Spiralschlange eines Schlangenwärmetauschers, um eine gemischte Lösung zu bilden, wobei der Carotinoidkristall in der suspendierten organischen Phase des Carotinoidkristalls in dem ersten organischen Lösungsmittel gelöst wird, um eine Ölphasenmatrix der Carotinoidlösungs-Lösung zu erhalten, wobei die Temperatur innerhalb der Spiralschlange 50 °C bis 70 °C beträgt;
Schritt S2: Mischen der Wasserphasenmatrix der kolloidalen Lösung mit der Ölphasenmatrix der Carotinoidlösungs-Lösung und aufeinanderfolgendes Durchführen einer Emulgierung und Dispersion sowie einer Homogenisierungsbehandlung, um eine Carotinoid-Nanodispersion zu erhalten, wobei die Wasserphasenmatrix der kolloidalen Lösung ein polymeres Schutzkolloid, einen kleinmolekularen Träger, ein zweites Antioxidans und Wasser umfasst und das zweite Antioxidans ein wasserlösliches Antioxidans ist;
Schritt S3: Entfernen des organischen Lösungsmittels aus der Carotinoid-Nanodispersion und Konzentrieren der Feuchtigkeit, um eine Carotinoid-Emulsion zu erhalten; und
Schritt S4: aufeinanderfolgendes Unterziehen der Carotinoid-Emulsion einer Sprühgranulierung und Trocknung, um das Carotinoidmittel zu erhalten;
wobei die Temperatur der suspendierten organischen Dispersionsphase des Carotinoidkristalls 15 °C bis 30 °C beträgt und die Temperatur des ersten organischen Lösungsmittels 50 bis 70 °C beträgt;
wobei die Verweilzeit der gemischten Lösung im Schlangenwärmetauscher 40 Sekunden bis 60 Sekunden beträgt;
wobei das Längenverhältnis der Spiralschlange 500 bis 2.000 : 1 beträgt; der Innendurchmesser der Spiralschlange 0,5 cm bis 2 cm beträgt und die Länge 5 m bis 20 m beträgt.

2. Herstellungsverfahren nach Anspruch 1, wobei der Massenanteil des Carotinoidkristalls in der suspendierten organischen Dispersionsphase des Carotinoidkristalls 20 % bis 40 % beträgt;
und/oder der Massenanteil der All-Trans-Formen im Carotinoidkristall größer oder gleich 95 % ist;
und/oder das Massenverhältnis des ersten organischen Lösungsmittels zum Carotinoidkristall 7,5 bis 20 : 1 beträgt.

3. Herstellungsverfahren nach Anspruch 1, wobei die suspendierte organische Dispersionsphase des Carotinoidkristalls ferner ein erstes Antioxidans umfasst, das ausgewählt ist aus mindestens einem von Dibutylhydroxytoluol (BHT), Butylhydroxyanisol (BHA), tert-Butylhydrochinon (TBHQ), Vitamin-C-Palmitat, Natriumascorbat, Ethoxychinolin und dl-α-Tocopherol.

4. Herstellungsverfahren nach Anspruch 3, wobei das Massenverhältnis des ersten Antioxidans zum Carotinoidkristall 0,05 bis 0,2 : 1 beträgt.

5. Herstellungsverfahren nach Anspruch 1, wobei das Lösungsmittel für die suspendierte organische Phase des Carotinoidkristalls ein zweites organisches Lösungsmittel ist.

6. Herstellungsverfahren nach Anspruch 5, wobei das erste organische Lösungsmittel und das zweite organische Lösungsmittel jeweils unabhängig voneinander ausgewählt sind aus mindestens einem von Dichlormethan, Tetrahydrofuran, Aceton, n-Hexan, Dimethylcarbonat, Ethylacetat, Isopropylacetat und Methyl-tert-butylether, vorzugsweise Dichlormethan.

7. Herstellungsverfahren nach Anspruch 1, wobei das Material der Spiralschlange Edelstahl oder Kupferrohr ist und/oder der Schlangenwärmetauscher ferner einen Mantel umfasst, der außerhalb der Spiralschlange vorgesehen ist und zum Einleiten eines Heizmediums verwendet wird.

8. Herstellungsverfahren nach Anspruch 1, wobei in Schritt S2 das Massenverhältnis der Wasserphasenmatrix der kolloidalen Lösung zur Ölphasenmatrix der Carotinoidlösungs-Lösung 1,5 bis 6 : 1 beträgt;
und/oder wobei die Homogenisierungsbehandlung unter Druck durchgeführt wird, mit einem Druck im Bereich von 20 MPa bis 60 MPa, vorzugsweise von 35 MPa bis 45 MPa.

9. Herstellungsverfahren nach Anspruch 1, wobei in Schritt S2 das Massenverhältnis des polymeren Schutzkolloids, des kleinmolekularen Trägers, des zweiten Antioxidans und des Wassers in der Wasserphasenmatrix der kolloidalen Lösung 10 bis 70 : 0 bis 60 : 0,5 bis 3 : 100 beträgt;
und/oder wobei das polymere Schutzkolloid ausgewählt ist aus mindestens einem von modifizierter Stärke, Gelatine, Lignosulfonat, Natriumkaseinat, Sojaprotein, Gummi arabicum oder Xanthangummi, vorzugsweise modifizierter Stärke oder Lignosulfonat;
und/oder wobei der kleinmolekulare Träger ausgewählt ist aus mindestens einem von Saccharose, Glucose, Fructose, Trehalose, Maltodextrin, resistentem Dextrin, Xanthodextrin, Sorbitol oder Mannitol; und/oder wobei das zweite Antioxidans ausgewählt ist aus mindestens einem von Vitamin C, Natriumvitamin C oder Natriumisovitamin C.

10. Herstellungsverfahren nach Anspruch 1, wobei in Schritt S3 die Viskositätszahl der Carotinoidemulsion 100 cp bis 2.000 cp beträgt.

11. Herstellungsverfahren nach einem der Ansprüche 1 bis 10, wobei in Schritt S4 die Sprühgranulierung in einer Granuliersäule durchgeführt wird, die einen Stärkestrom enthält;
und/oder wobei Schritt S4 ferner einen Stärketrennschritt nach dem Trocknen umfasst, wobei vorzugsweise das Trocknen und die Stärketrennung jeweils unabhängig voneinander in einem Fließbett durchgeführt werden;
und/oder wobei die Sprühgranulierung das Sprühen der Carotinoidemulsion in die Granuliersäule, die einen Stärkestrom enthält, durch einen Zentrifugalsprüher umfasst, wobei die Lufteintrittstemperatur der Granuliersäule vorzugsweise 100 °C bis 130 °C beträgt und die Drehzahl des Zentrifugalsprühers vorzugsweise 8.000 U/min bis 15.000 U/min beträgt;
und/oder wobei der Gewichtsverlust beim Trocknen des Carotinoidmittels weniger als oder gleich 8 Gew.-% beträgt.

12. Herstellungsverfahren nach einem der Ansprüche 1 bis 11, wobei der Carotinoidkristall ausgewählt ist aus mindestens einem von β-Carotinkristallen, Canthaxanthinkristallen, Astaxanthinkristallen, Luteinkristallen, Lycopenkristallen, Apoesterkristallen oder Zeaxanthinkristallen.

## Revendications

1. Procédé de préparation d'un agent caroténoïde, dans lequel, le procédé de préparation comprend
étape S1, le mélange d'une phase de dispersion organique en suspension d'u cristal caroténoïde avec un premier solvant organique dans une bobine en spirale d'un échangeur de chaleur à bobine pour former une solution mixte, avec le cristal caroténoïde dans la phase organique en suspension du cristal caroténoïde étant dissous dans le premier solvant organique pour obtenir une matrice de phase huileuse de la solution de dissolution de caroténoïde, la température à l'intérieur de la bobine en spirale étant de 50 °C à 70 °C ;
étape S2, le mélange de la matrice de phase aqueuse de la solution colloïdale avec la matrice de phase huileuse de la solution de dissolution de caroténoïde et effectuer l'émulsification et la dispersion ainsi qu'un traitement d'homogénéisation successivement pour obtenir une nanodispersion de caroténoïde, la matrice de phase aqueuse de la solution colloïdale comprenant un colloïde protecteur polymère, un petit support moléculaire, un second antioxydant et de l'eau et le second antioxydant est un antioxydant hydrosoluble,
étape S3, l'enlèvement du solvant organique de la nanodispersion de caroténoïde et la concentration de l'humidité pour obtenir une émulsion de caroténoïde ; et
étape S4, la soumission de l'émulsion de caroténoïde à une granulation par pulvérisation et un séchage successivement pour obtenir l'agent caroténoïde,
la température de la phase de dispersion organique en suspension du cristal caroténoïde est de 15 °C à 30 °C et la température du premier solvant organique est de 50 à 70 °C ;
le temps de résidence de la solution mixte dans l'échangeur de chaleur à bobine est de 40 secondes à 60 secondes,
le rapport d'aspect de la bobine en spirale est de 500 à 2 000 : 1 ; le diamètre interne de la bobine en spirale est de 0,5 à 2 cm et la longueur est de 5 m à 20 m.

2. Procédé de préparation selon la revendication 1, dans lequel, la teneur en masse du cristal caroténoïde dans la phase de dispersion organique en suspension du cristal caroténoïde est de 20 % à 40 % ;
et/ou, la teneur en masse de toutes les formes trans dans le cristal caroténoïde est supérieure ou égale à 95 %,
et/ou, le rapport en masse du premier solvant organique au cristal caroténoïde est de 7,5 à 20 : 1.

3. Procédé de préparation selon la revendication 1, dans lequel, la phase de dispersion organique en suspension du cristal caroténoïde comprend en outre un premier antioxydant, qui est choisi parmi au moins un du dibutylhydroxytoluène (BHT), de l'hydroxyanisole butylé (BHA), de tert-butylhydroquinone (TBHQ), de palmitate de vitamine C, d'ascorbate de sodium, d'éthoxyquinoline, de dl-α tocophérol.

4. Procédé de préparation selon la revendication 3, dans lequel, le rapport en masse du premier antioxydant au cristal caroténoïde est de 0,05 à 0,2 : 1.

5. Procédé de préparation selon la revendication 1, dans lequel, le solvant pour la phase organique en suspension du cristal caroténoïde est un second solvant organique.

6. Procédé de préparation selon la revendication 5, dans lequel, le premier solvant organique et le second solvant organique sont chacun indépendamment choisi parmi au moins un du dichlorométhane, du tétrahydrofurane, de l'acétone, du n-hexane, du carbonate de diméthyle, de l'acétate d'éthyle, de l'acétate d'isopropyle et du méthyl tert-butyl éther, de préférence du dichlorométhane.

7. Procédé de préparation selon la revendication 1, dans lequel, le matériau de la bobine en spirale est un tube d'acier inoxydable ou de cuivre, et/ou, l'échangeur de chaleur à bobine comprend en outre une chemise, qui est fournie à l'extérieur de la bobine en spirale et est utilisée pour introduire un milieu de chaleur.

8. Procédé de préparation selon la revendication 1, dans lequel, dans l'étape S2, le rapport en masse de la matrice de phase aqueuse de la solution colloïdale à la matrice de phase huileuse de la solution de dissolution de caroténoïde est de 1,5 à 6 : 1 ;
et/ou, le traitement d'homogénéisation est réalisé sous pression, avec une pression se situant dans la plage allant de 20 MPa à 60 MPa, de préférence de 35 MPa à 45 MPa.

9. Procédé de préparation selon la revendication 1, dans lequel, dans l'étape S2, le rapport en masse du colloïde protecteur polymère, du petit support moléculaire, du second antioxydant et de l'eau dans la matrice de phase aqueuse de la solution colloïdale est de 10 à 70 : 0 à 60 : 0,5 à 3 : 100,
et/ou le colloïde protecteur polymère est choisi parmi au moins un de l'amidon modifié, de la gélatine, du lignosulfonate, du caséinate de sodium, de la protéine de soja, de la gomme arabique et de la gomme de xanthane, de préférence de l'amidon modifié ou du lignosulfonate,
et/ou, le petit support moléculaire est choisi parmi au moins un du saccharose, du glucose, du fructose, du tréhalose, de la maltodextrine, de la dextrine résistante, de la xanthodextrine, du sorbitol et du mannitol et/ou, le second antioxydant est choisi parmi au moins une de la vitamine C, de la vitamine C de sodium ou de l'isovitamine C de sodium.

10. Procédé de préparation selon la revendication 1, dans lequel, dans l'étape S3, le nombre de viscosité de l'émulsion de caroténoïde est de 100 cp à 2 000 cp.

11. Procédé de préparation selon l'une quelconque des revendications 1 à 10, dans lequel, dans l'étape S4, la granulation par pulvérisation est réalisée dans une colonne de granulation contenant un écoulement d'amidon,
et/ou l'étape S4 comprend en outre une étape de séparation d'amidon après le séchage, de préférence le séchage et la séparation d'amidon sont réalisés chacun indépendamment dans un lit fluidisé,
et/ou, la granulation par pulvérisation comprend la pulvérisation de l'émulsion de caroténoïde contenant un écoulement d'amidon par un pulvérisateur à centrifugeuse, la température d'entrée d'air de la colonne de granulation est de préférence de 100 °C à 130 °C, la vitesse rotationnelle du pulvérisateur à centrifugeuse est de préférence de 8 000 t/min à 15 000 t/min,
et/ou, la perte de poids au séchage de l'agent caroténoïde est inférieure ou égale à 8 % en poids.

12. Procédé de préparation selon l'une quelconque des revendications 1 à 11, dans lequel, le cristal caroténoïde est choisi parmi au moins un des cristaux β-carotène, des cristaux canthaxanthine, des cristaux astaxanthine, des cristaux lutéine, des cristaux lycopène, des cristaux apoester ou des cristaux zéaxanthine.
